# EUROPEAN PATENT SPECIFICATION

(11) **EP 2 289 569 B1**
(45) Date of publication and mention of the grant of the patent: **22.03.2017**
(21) Application number: 10179802.3
(22) Date of filing: 08.02.2005
(51) Int. Cl.: A61L 27/24, A61L 27/56, A61L 31/04, A61L 31/14

(54) **Collagen device and method of preparing the same**
Kollagenartikel und Verfahren zu dessen Herstellung
Dispositif de collagène et son procédé de préparation

(30) Priority: 09.02.2004 US 542968 P; 27.04.2004 US 565747 P; 30.09.2004 US 955835
(43) Date of publication of application: 02.03.2011
(62) Divisional of application: 05250694.6
(73) Proprietor: CODMAN & SHURTLEFF INC., Rynham, MA 02767 (US)
(72) Inventor: Macomber, Laurel R., N. Attleboro, MA 02760 (US); Sommerich, Robert E., Norton, MA 02766 (US); Shenoy, Vivek N., Sunnyvale, CA 94086 (US); Halvorsen, Matthew J., Hopkinton, NJ 03229 (US)
(74) Representative: Kirsch, Susan Edith

(56) References cited:
- EP-A- 0 440 198
- WO-A1-99/13902
- US-A- 4 066 083
- US-A- 5 206 028
- US-A- 5 997 895
- US-A1- 2003 133 967

## Description

### FIELD OF THE INVENTION

The present invention relates to a collagen device . More specifically, the present invention relates to a collagen device for use as an implant to replace, reinforce or strengthen bodily tissue, an adhesion barrier, or for use as a short-term body contact for moisture retention, hemostasis or tissue protection.

### BACKGROUND OF THE INVENTION

The human brain and spinal cord are covered with meningeal membranes whose integrity is critical to the operation of the central nervous system. When the integrity of a person's meningeal membranes is intentionally or accidentally compromised, serious consequences may ensue, unless the membranes can be repaired.

The meningeal membrane comprises three overlapping layers of tissue, which are in order from outside to inside, the dura mater (or dura), the arachnoid and the pia mater. Repairing damaged meningeal membranes has largely focused on implantable and/or resorbable constructs (known as dural substitutes) which are grafted to the damaged dura mater and are designed to replace and/or regenerate the damaged tissue.

The prior art in the field of the present invention includes US 5 997 895 A, which relates to the repair of damaged tissue, and more specifically, to the use of non-infectious collagen to heal damaged dural tissue. In addition, WO99/13902 A1 discloses dural substitutes, which include collagen treated and/or prepared to be physiologically compatible and substantially free of active viruses and prions. US2003/133967 discloses a multilayer material with improved mechanical, physical, functional and handling properties for use in human and veterinary medicine for reconstruction of tissue or organs comprises at least two layers of a reconstituted natural polymer matrix, preferentially collagen, and optionally one or more layers comprising a biocompatible synthetic polymer which may be bioinert or biodegradable. EP 0 440 198 A2 provides a process for preparing collagen sponge, the process comprising the steps of impregnating cross-linked collagen sponge with an aqueous solution of a hydrophilic organic solvent, freezing the sponge and vacuum-drying the sponge. Furthermore, US 5 206 028 A discloses collagen membranes having physical and biological properties which make them suitable medical uses, particularly as a periodontal barrier.

### SUMMARY OF THE INVENTION

The present invention provides a collagen device for use as a dural substitute, said collagen device comprising: a cross-linked collagen sheet, said sheet having a tensile strength of about 58.6 kPa (8.5 psi) or greater as defined by the present set of claims.

Suitably, the cross-linked collagen sheet is obtainable by a process comprising the steps of:
(i) mixing collagen with purified water for a period of time sufficient to form a mixture;
(ii) lyophilizing the mixture into a collagen sheet; and
(iii) cross-linking the collagen sheet.

In embodiments, the collagen device consists of said cross-linked collagen sheet.

Surprisingly, the collagen device of the present invention has good handling properties, as the collagen device is sufficiently flexible to conform to irregular-shaped surfaces but stiff enough that it does not curl or adhere to itself, instruments or the practitioner's gloved hands when wet. In addition, the collagen device in accordance with the present invention has very good strength properties, such as tensile strength, making it very easy to handle for the physician. Further, the collagen device in accordance with the present invention can be the same shape or size as conventional collagen devices, such as currently available collagen dural grafts, while still providing the surgeon with a device that has superior strength and handling properties.

The collagen device in accordance with the present invention is substantially fully resorbable, despite having a majority of its pores having a diameter of less than 10µm. Surprisingly, the present inventors have found that despite the fact that those skilled in the art believe that the pore size must be sufficiently large enough (150µm pore diameter is preferred for internal pores and 70µm is preferred for surface pores) to permit growing meningeal tissue to infiltrate therein, the present invention collagen is replaced by growing meningeal tissue and is substantially fully resorbable even though a majority of its pores have a diameter of less than 10µm.

The collagen device of the present invention can be prepared by mixing collagen with purified water for a period of time sufficient to form a mixture. The pH of the mixture is adjusted to a pH level sufficient to substantially solubilize the collagen. A first predetermined amount of the mixture is placed into a container. The mixture is subject to a lyophilizing process and formed into a collagen device. The collagen device is also cross-linked. The collagen device has a plurality of pores wherein a majority of the pores have a diameter of less than 10µm. To use the collagen device as an implant to replace, reinforce or strengthen bodily tissue, or to act as an adhesion barrier, the collagen device is placed in contact with bodily tissue and that contact is maintained until the collagen device is substantially resorbed within the bodily tissue.

### BRIEF DESCRIPTION OF THE DRAWINGS

The invention can be more fully understood from the following detailed description taken in conjunction with the accompanying drawings, in which:
FIG. 1 is a flow chart illustrating a method of preparing a collagen device in accordance with the present invention;
FIGS. 2A, 2B and 2C are a lower perspective view, side view and top view respectively of a collagen device; and
FIGS. 3A-3C show a collagen device made of a multi-layer or laminate product.

### DETAILED DESCRIPTION OF THE INVENTION

It will be understood that the foregoing is only illustrative of the principles of the invention, and that various modifications can be made by those skilled in the art without departing from the scope of the invention, as defined in the appended claims.

A collagen device in accordance with the present invention is prepared by mixing collagen with purified water for a period of time sufficient to form a mixture. The ratio of collagen to purified water is between approximately 0.4% to 5.0% w/w. The pH of the mixture is then adjusted to a pH level sufficient to substantially solubilize the collagen. A predetermined amount of the mixture is then placed into a container. The mixture is then formed into a collagen sheet by a lyophilizing process. The mixture could also be formed into a block, cylinder, or other desired shape, which will hereinafter be referred to collectively as a collagen sheet. The collagen sheet is then cross-linked. During the cross-linking, the collagen sheet is preferably exposed to a liquid or vapor form of a cross-linking agent, such as formaldehyde or glutaraldehyde. Thereafter, the collagen sheet is ventilated if the cross-linking agent is vapor or relyophilized if it is liquid. The steps of forming the mixture into a collagen sheet and the cross-linking could be reversed.

The resulting collagen sheet has a plurality of pores wherein a majority of the pores have a diameter of less than 10µm. Preferably, greater than 80% of the pores have a diameter of less than 10µm. More preferably, greater than 90% of the pores have a diameter of less than 10µm. Even more preferably, greater than 95% of the pores have a diameter of less than 10µm. Yet even more preferably, greater than 98% of the pores have a diameter of less than 10µm. And even more preferably, approximately all of the pores have a diameter of less than 10µm.

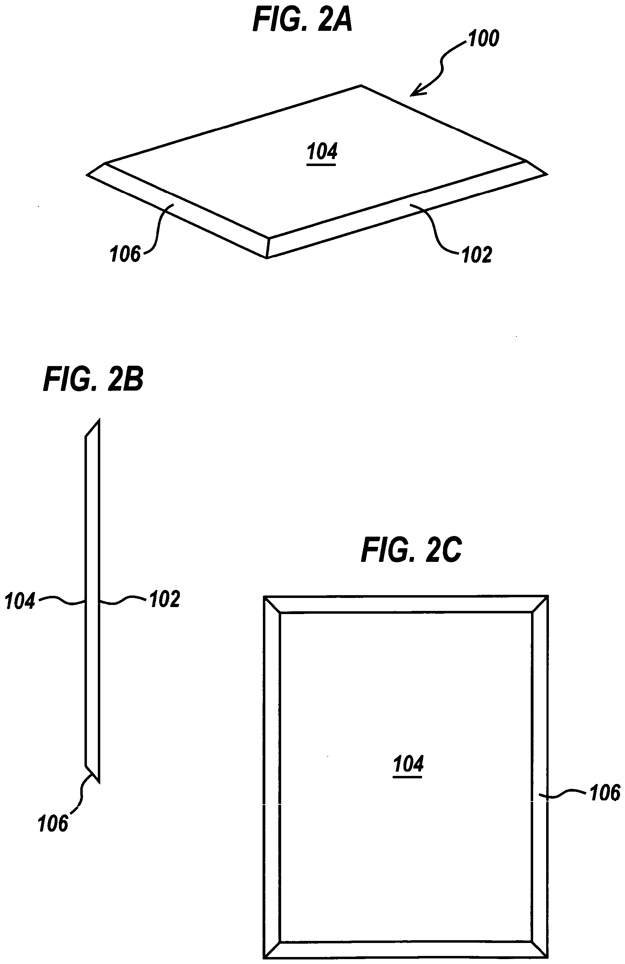

The collagen sheet 100 may be cut into predetermined shapes or formed in predetermined shapes that are formed to size. Sheet 100 has a top surface 102, bottom surface 104 and peripheral edge 106. The edge 106 of each predetermined shape may be chamfered to allow a smooth profile of the edge when it is wetted *in situ*, as shown in Figs. 2A-2C. The angle of the chamfer D is preferably approximately 30 to 75 degrees from vertical pivoting from the top or bottom surface.

Alternatively, before cross-linking, the collagen sheet can be compressed by rollers. The collagen sheet can be compressed to between approximately one-half to one-eighths the original thickness C of the collagen sheet.

In use, for use as a dural substitute or adhesion barrier, or for short-term body contact for moisture retention, hemostasis, or tissue protection, the collagen sheet may be placed in contact with bodily tissue. When used as an implant, contact between the collagen sheet and the bodily tissue is maintained. In time, currently estimated to be about nine (9) months, the collagen sheet will be fully resorbed. When placing the collagen sheet in contact with bodily tissue, the collagen sheet does not stick to or adhere to instruments, including the surgeon's hands. Also, should the collagen sheet need to be repositioned, the surgeon is able to do so without the collagen sheet breaking apart

The collagen sheet has very good strength properties, such as tensile strength, making it very easy to handle for the physician. In testing done in accordance with ASTM 638, Type V, the collagen sheet of the present invention had an average tensile strength greater than 41.4 kPa (6.0 psi) ranging from 51.2 kPa (7.43 psi) to 67.3 kPa (9.76 psi) per lot, with an average of about 60.3 kPa (8.74 psi) for all lots tested. Currently available collagen sheets were tested and they had an average tensile strength of about 41.4 kPa (6.00 psi).

One skilled in the art will readily recognize that the collagen device described herein can also be used to deliver biologically active agents such as, for example, growth factors, autologous cells, bone marrow, antibiotics, anti-cancer agents, and gene and DNA constructs.

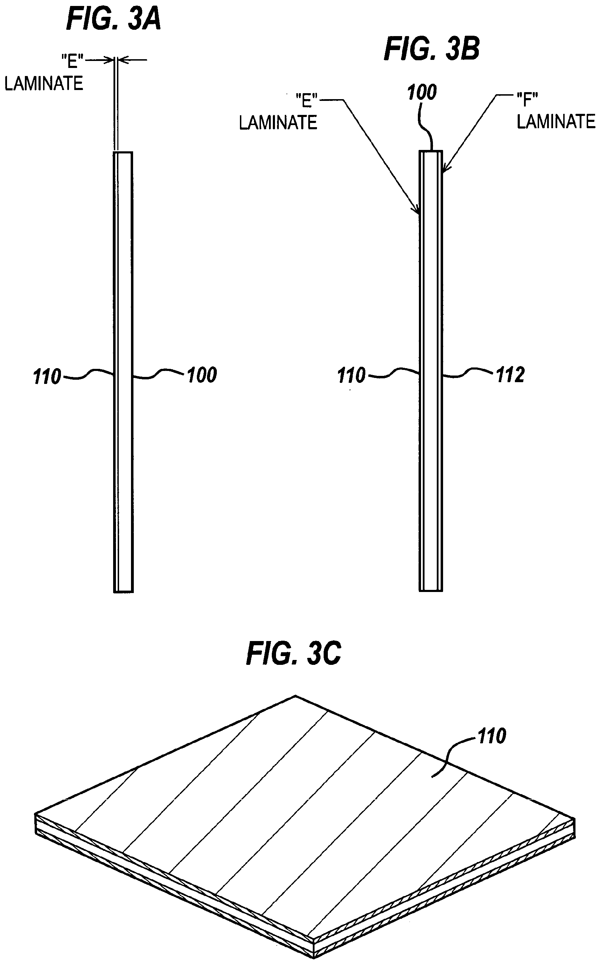

The collagen device may be used to provide a component of a multi-layer or laminate product, as illustrated in Figs 3A-C. The collagen sheet 100 can include one or more layers or laminates 110, 112 as shown (Fig 3A shows one laminate, and Figs 3B and 3C show two laminates). The collagen sheet described can be laminated or otherwise attached with one or a number of the following: film, felt, woven or non-woven matrix, mesh or a second collagen sheet. For example, a collagen sheet as described may be combined with an impermeable film to provide a watertight construct. The final multi-layer construct would be manufactured in order to improve one or a number of the following characteristics: suture retention strength, fluid impermeability, resorption duration, handling characteristics, stiffness, and/or adhesion properties to tissues.

The collagen sheet may include a layer of a film or woven matrix at the time of processing the collagen sheet so that it is incorporated within the boundaries of the collagen sheet. An alternate method would be to apply the second layer to the collagen sheet by various methods including but not limited to adhesives, heat-pressing, and combining layers during partial processing of one or both materials. The laminate or multi-layer product can include any biocompatible materials that may or may not be resorbable. In addition, the layer added to the collagen device may have biological active agents (e.g., antibiotics, growth factors, hemostasis factors, anti-cancer agents) incorporated within or upon the material while it may or may not be on the collagen device.

The various dimensions of the laminate structures may vary from matching dimensions to one or multiple layers have greater or smaller dimensions than one of the other layers. In this manner, the preferential characteristics of one layer may be emphasized at a certain location as desired, depending upon the requirements of the surgical procedure.

### EXAMPLE

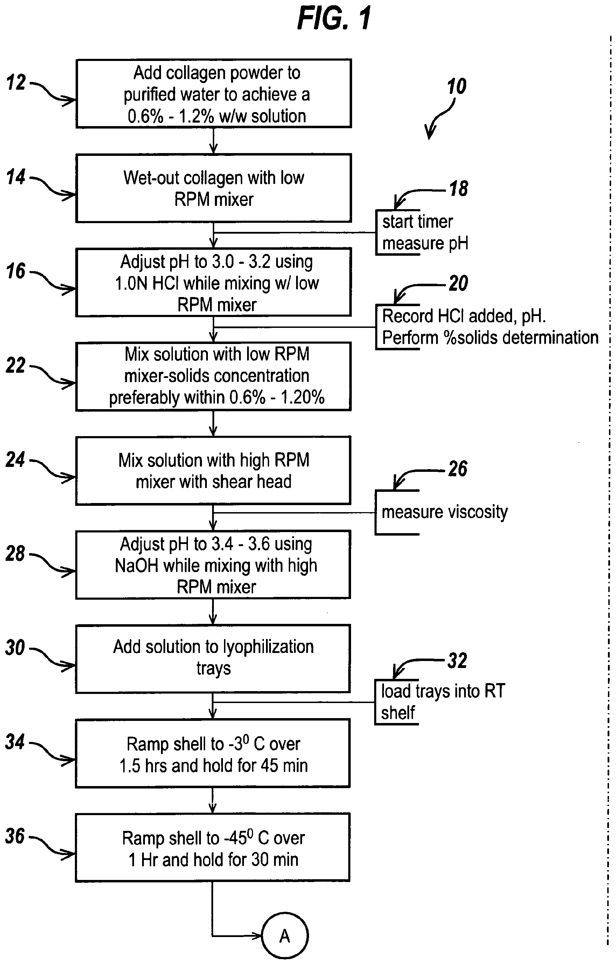

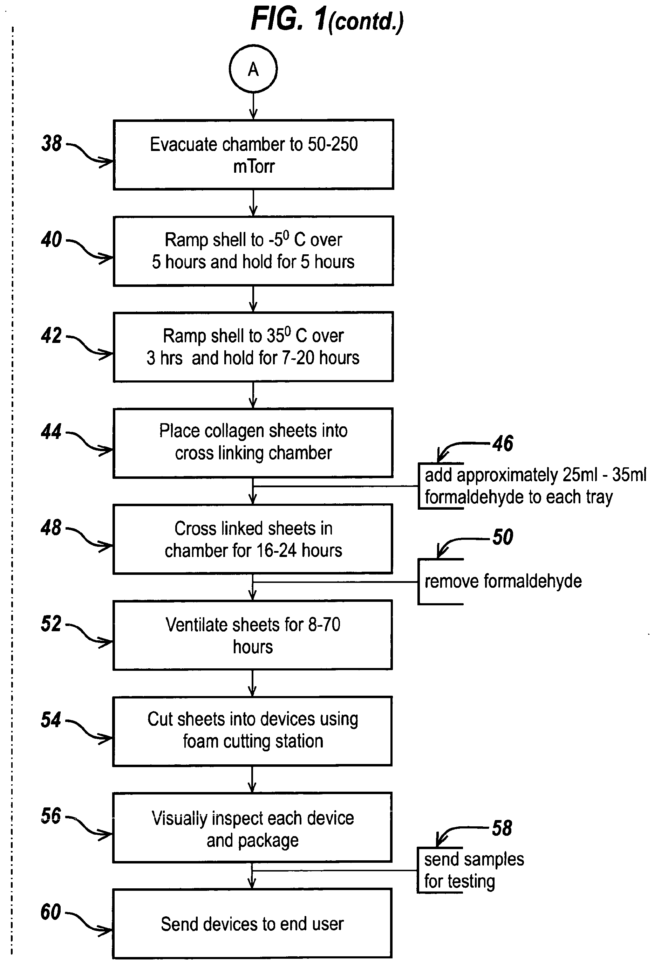

Referring now to Fig. 1, a non-limiting example of a collagen device made in accordance with method 10 for preparing a collagen device in accordance with the present invention is illustrated. The method includes a first step 12 of adding a collagen powder to purified water preferably in a ratio of approximately 0.4% to 5.0% w/w of collagen powder to purified water to hydrate the collagen powder. A ratio of about 0.40% to about 3.50% w/w is even more preferred. While a ratio of about 0.60% to about 1.20% w/w is most preferred.. The collagen powder is commercially available from Datascope of 14 Phillips Parkway, Montvale, New Jersey.

The hydrated collagen is then mixed in step 14 with the purified water for a period of time sufficient to form a mixture. This period of time is preferably from about three (3) to six (6) minutes. The mixing is preferably achieved first with a relatively gentle mixer sufficient to solubilize the collagen with minimal or no shearing of the collagen fibers. This gentle mixer may be a Lightnin™ mixer model L1U03 that mixes at 0 to 1000 rpm and is commercially available from Lightnin, which is a unit of General Signal of Coolock Dublin, Ireland.

During the mixing, the pH of the mixture is adjusted to a predetermined pH level in step 16. The predetermined pH level is preferably between approximately 1.5 and 4.0, which is below the isoelectric point of the mixture. Alternatively , the predetermined pH level is preferably between approximately 11.0 and 13.5, which is above the isoelectric point of the mixture. At the initiation of the adjusting of the pH, a timer is initiated, as illustrated in step 18. The pH of the mixture is preferably achieved while the mixture is being mixed with the gentle mixer at a mixing speed of between about 400 and 1000 rpm to a pH of about 3.0-3.2. To adjust the pH, 1.0N HCl is preferably added to the mixture. Of course, while hydrochloric acid is preferably used to adjust the pH of the mixture, other acids may be used, such as, for example, acetic acid, lactic acid, or phosphoric acid.

The adjusting the pH step is preferably achieved without overshooting the predetermined pH level. If one were to overshoot the pH level, then an additive such as NaOH would have to be added to the mixture to raise the pH level. Sodium hydroxide is preferably used to adjust the pH of the collagen solution, although other hydroxides may be used, such as, for example, other alkali metal hydroxides or ammonium hydroxides. But the present inventors have discovered that the raising and lowering or lowering and raising of the pH of the mixture may cause inconsistent freezing which may affect the desired pore size and biocompatibility due to the change in ionic strength. Thus, it is preferred not to overshoot the predetermined pH level. During the adjusting step 16, the amount of HCl added to the mixture, the pH, and a calculation of the percentage of the solids concentration is determined, as illustrated in step 20.

Once the predetermined pH level is achieved in step 16, the mixture is continued to be mixed with the gentle mixer for preferably at least one (1) hour total elapsed time from the time the powder was added to the purified water in step 12, as illustrated in step 22. The percentage of solids concentration is preferably within 0.6% - 1.2%.

After mixing with the gentle mixer, the mixture is mixed with a shear mixer preferably at a mixing speed of between about 8000 and 9000 rpm, as illustrated in step 24. The shear mixture preferably operates at a speed that is sufficient to mechanically break down the collagen powder. This shear mixer may be a Silverson™ mixer that mixes at 0 to 10,000 rpm and is commercially available from Silverson Machines Limited of Waterside Chesham Bucks, England. The pH of the mixture is preferably further adjusted while the mixture is being mixed with the shear mixer to a pH of about 3.4-3.6.

The viscosity of the mixture is measured in step 26 preferably with the initiation of mixing step 24.

The pH is raised to improve sheet handling properties. This adjustment is preferably achieved without overshooting the predetermined pH level. If one were to overshoot the pH level, then an additive such as HCl would have to be added to the mixture to lower the pH level.

Once step 28 is complete, a predetermined amount of the mixture is placed into a container, as illustrated in step 30. A sufficient amount of the mixture is placed into the container so that the resultant collagen device will have sufficient thickness to perform as a dural substitute, adhesion barrier, or for short-term body contact for moisture retention, hemostasis, or tissue protection. The tray is preferably made of a plastic material, such as PETG. However, the trays could be made from glass, metal, ceramic, a base material coated with a non-stick surface such as TEFLON® or polished metal. The trays could also be shaped with individual compartments with each compartment shaped to the desired final form of the collagen device. For example, the compartments can be of 1" x 1" square, with beveled edges on each edge. Of course, many different sizes or shapes could be made with or without beveled edges, including within the same tray, to meet the needs of the surgeon.

The container is placed in a chamber,as illustrated in step 32. The container can be placed on a shelf within the chamber, and the shelf has a temperature control mechanism to control the temperature of the shelf, and thereby the chamber. Hereinafter, the temperature of the chamber will be referred to, but one skilled in the art will recognize that this includes the temperature of the shelf. The temperature control mechanism is regulated so that the temperature of the chamber is preferably above the crystallization temperature of the mixture. The bottom surface of the container is preferably planer to mate with the planer surface of the top surface of the shelf.

The temperature of the chamber can be at room temperature, which is between about 15 to 25°C. Alternatively the chamber can be about-3°C. Alternatively, the chamber temperature can be set well below the crystallization temperature of the mixture to about -50°C to deep freeze the mixture upon placement into the chamber. If the temperature is at room temperature, then the temperature of the chamber is adjusted to a second predetermined temperature approximately slightly above the crystallization temperature of the mixture over approximately a first predetermined time period, as illustrated in step 34. Preferably, the second predetermined temperature is -3°C to -5°C, and the first predetermined time period is approximately sixty (60) minutes. The chamber is then held at the second predetermined temperature for approximately forty-five (45) minutes.

The temperature of the chamber is the cooled to approximately -45°C over a period of approximately one (1) hour, as illustrated in step 36. The chamber is preferably held at this approximate temperature for about at least thirty (30) minutes.

A vacuum is then pulled in the chamber to approximately a first predetermined level sufficient to allow adequate sublimation of ice crystals the chamber is evacuated, as illustrated in step 38. The vacuum can be pulled while the temperature of the chamber is being held at -45°C in step 34. The chamber can be evacuated to about 50 - 250 mTorr. Sublimation of the ice crystals results in the formation of a collagen sheet having a plurality of pores wherein a majority of the pores have a diameter of less than 10 µm.

The chamber temperature is then raised to a sufficient temperature and held at this temperature for a sufficient period of time until primary drying occurs in the mixture, as illustrated in step 40. The chamber can be ramped up to about -5°C over about five (5) hours and this temperature is maintained for about five (5) hours. In this non-limiting Example, the mixture is transformed by the above steps into a collagen sheet.

As illustrated in step 42, the temperature of the chamber is then changed to approximately room temperature over approximately seven (7) hours. The chamber can be raised to about 35°C over approximately three (3) hours and is held at this temperature for a sufficient period of time until secondary drying occurs in the collagen sheet without excessive drying or meltback, which can be for about seven (7) to twenty (20) hours.

Alternatively, the collagen sheet could be compressed by rollers or plates, as one skilled in the art will readily recognize. The rollers can compress the sheet to between one-half to less than 5% of the sheets original thickness. Compressing the sheet may result in a collagen sheet that is stronger than conventional sheets.

The collagen sheet is then placed in a cross-linking chamber, as illustrated in step 44. The sheets of collagen can be hung in the cross-linking chamber or placed on screens. Of course, the sheets could remain in the same chamber, and the cross-linking processing could take place in this chamber.

A predetermined amount of a cross-linking agent is added to the cross-linking chamber in step 46. The predetermined amount of formaldehyde is sufficient to at least partially saturate the collagen sheet. The cross-linking agent is formaldehyde, and the predetermined amount of formaldehyde can be between approximately 25ml and 35ml. (Of course, the amount of formaldehyde added is dependent on the number of sheets and size of the chambers). The collagen sheet is exposed to a liquid or vapor form of the cross-linking agent. The cross-linking agent is removed from the cross-linking chamber after approximately sixteen (16) and twenty-four (24) hours in steps 48 and 50.

The collagen sheet is preferably cross-linked by vapor cross-linking or solution cross-linking. If a solution is used, the sheet is preferably dehydrated by lyophilization. Cross-linking agents such as formaldehyde, glutaraldehyde, carbodiimides or difunctional succinimides may be used. Alternatively, the matrix may be cross-linked by dehydrothermal cross-linking or UV radiation.

The collagen sheets are ventilated for between approximately eight (8) and seventy (70) hours in step 52, to remove excess cross-linking agent.

The collagen sheet is then cut into the desired shapes at a cutting station in step 54. The collagen sheet may be formed in predetermined shapes that are formed to size within the tray. The edge of each predetermined shape may be chamfered to allow a smooth profile of the edge when it is wetted *in situ.* The angle of the chamfer is preferably approximately 30 to 75 degrees from vertical.

Each cut section of the collagen sheet is then inspected, preferably visually, in step 56. Thereafter, some samples can be sent for testing in step 58 and the remaining cut sections can be packaged in a conventional manner sterilized and then sent to the end user, in step 60. The collagen sheet is tested, preferably by test method ASTM E1294, to ensure that the porosity of the sheet is less than 10µm in step 58.

The steps of cutting the collagen sheet into the desired shapes and the cross-linking could be reversed.

One skilled in the art will appreciate further features and advantages of the invention based on the above-described embodiments. While there have been shown, described, and pointed out fundamental novel features of the invention as applied to a preferred embodiment thereof, it will be understood that various omissions, substitutions, and changes in the form and details of the devices illustrated, and in their operation, may be made by those skilled in the art without departing from the scope of the invention as defined in the appended claims. For example, it is expressly intended that all combinations of those elements and/or steps which perform substantially the same function, in substantially the same way, to achieve the same results are within the scope of the invention. Substitutions of elements from one described embodiment to another are also fully intended and contemplated. It is also to be understood that the drawings are not necessarily drawn to scale, but that they are merely conceptual in nature. Accordingly, the invention is not to be limited by what has been particularly shown and described, except as indicated by the appended claims.

## Claims

1. A collagen device for use as a dural substitute, said collagen device comprising a cross-linked collagen sheet, said sheet having a tensile strength of about 58.6kPa (8.5 psi) or greater, said sheet having a plurality of pores, a majority of said pores having a diameter of less than 10 micrometers.

2. The collagen device of claim 1, wherein the tensile strength is about 58.6kPa (8.5 psi).

3. The collagen device of claim 1 or 2, wherein greater than 95% of the device pores have a diameter of less than 10 micrometers.

4. The collagen device of claim 1 or 2, wherein greater than 98% of the device pores have a diameter of less than 10 micrometers.

5. The collagen device of claim 1 or 2, wherein approximately all of the device pores have a diameter of less than 10 micrometers.

6. The collagen device of any preceding claim, wherein the collagen is recombinant collagen.

7. The collagen device of any preceding claim, wherein the collagen device contains a biological active agent.

8. The collagen device of any preceding claim, further comprising, attached to or incorporated within the sheet, at least one of a film, a felt, a matrix, a mesh or a second collagen sheet.

9. The collagen device of claim 8, wherein a fluid impermeable film is attached to, or is incorporated within, the sheet.

10. The collagen device of claim 8 or 9, wherein either of the film, the felt, the matrix, the mesh or the second collagen sheet contains a biological active agent.

11. The collagen device of claim 10, wherein the first collagen sheet contains a biological active agent, and the film, the felt, the matrix, the mesh or the second collagen sheet also contains a biological active agent, and the biological active agent contained in the first sheet is different from the biological active agent contained in either the film, the felt, the matrix, the mesh or the second collagen sheet.

12. The collagen device of claim 10, wherein the first collagen sheet contains a biological active agent, and the film, the felt, the matrix, the mesh or the second collagen sheet also contains a biological active agent, and the biological active agent contained in the first sheet is the same as the biological active agent contained in either the film, the felt, the matrix, the mesh or the second collagen sheet.

13. The collagen device of any one of claims 7 or 10-12, wherein the biological active agent is at least one growth factor an antibiotic or a combination of antibiotics, or an anti-cancer agent or a combination of anti-cancer agents.

14. The collagen device of any preceding claim, wherein said cross-linked collagen sheet is obtainable by a process comprising the steps of:
(i) mixing collagen with purified water for a period of time sufficient to form a mixture;
(ii) lyophilizing the mixture into a collagen sheet; and
(iii) cross-linking the collagen sheet.

15. The collagen device of any one of claims 1 to 7, wherein the collagen device consists of said cross-linked collagen sheet.

## Patentansprüche

1. Kollagenartikel zur Verwendung als Duraersatz, wobei der Kollagenartikel eine vernetzte Kollagenlage umfasst, die Lage eine Zugfestigkeit von etwa 58,6 kPa (8,5 psi) oder mehr aufweist, die Lage eine Vielzahl von Poren aufweist, wobei ein überwiegender Teil der Poren einen Durchmesser kleiner als 10 Mikrometer aufweist.

2. Kollagenartikel nach Anspruch 1, wobei die Zugfestigkeit etwa 58,6 kPa (8,5 psi) beträgt.

3. Kollagenartikel nach Anspruch 1 oder 2, wobei mehr als 95 % der Poren des Artikels einen Durchmesser von weniger als 10 Mikrometer aufweisen.

4. Kollagenartikel nach Anspruch 1 oder 2, wobei mehr als 98 % der Poren des Artikels einen Durchmesser von weniger als 10 Mikrometer aufweisen.

5. Kollagenartikel nach Anspruch 1 oder 2, wobei im Wesentlichen alle der Poren des Artikels einen Durchmesser von weniger als 10 Mikrometer aufweisen.

6. Kollagenartikel nach einem der vorhergehenden Ansprüche, wobei das Kollagen rekombinantes Kollagen ist.

7. Kollagenartikel nach einem der vorhergehenden Ansprüche, wobei der Kollagenartikel einen biologischen Wirkstoff enthält.

8. Kollagenartikel nach einem der vorhergehenden Ansprüche, der ferner daran angebracht oder in die Lage eingebaut mindestens eines von einem Film, einem Filz, einer Matrix, einem Maschenwerk oder einer zweiten Kollagenlage umfasst.

9. Kollagenartikel nach Anspruch 8, wobei ein fluidundurchlässiger Film an der Lage angebracht oder in diese eingebaut ist.

10. Kollagenartikel nach Anspruch 8 oder 9, wobei jeder von dem Film, dem Filz, der Matrix, dem Maschenwerk oder der zweiten Kollagenlage einen biologischen Wirkstoff enthält.

11. Kollagenartikel nach Anspruch 10, wobei die erste Kollagenlage einen biologischen Wirkstoff enthält und der Film, der Filz, die Matrix, das Maschenwerk oder die zweite Kollagenlage ebenfalls einen biologischen Wirkstoff enthält, und wobei der in der ersten Lage enthaltene biologische Wirkstoff sich von dem biologischen Wirkstoff unterscheidet, der in einem von dem Film, dem Filz, der Matrix, dem Maschenwerk oder der zweiten Kollagenlage enthalten ist.

12. Kollagenartikel nach Anspruch 10, wobei die erste Kollagenlage einen biologischen Wirkstoff enthält und der Film, der Filz, die Matrix, das Maschenwerk oder die zweite Kollagenlage ebenfalls einen biologischen Wirkstoff enthält, und wobei der in der ersten Lage enthaltene biologische Wirkstoff der gleiche wie der biologische Wirkstoff ist, der in einem von dem Film, dem Filz, der Matrix, dem Maschenwerk oder der zweiten Kollagenlage enthalten ist.

13. Kollagenartikel nach einem der Ansprüche 7 oder 10-12, wobei der biologische Wirkstoff mindestens ein Wachstumsfaktor, ein Antibiotikum oder eine Kombination von Antibiotika, oder ein Antikrebsmittel oder eine Kombination von Antikrebsmitteln ist.

14. Kollagenartikel nach einem der vorhergehenden Ansprüche, wobei die vernetzte Kollagenlage nach einem Verfahren erhältlich ist, das die folgenden Schritte umfasst:
(i) Mischen von Kollagen mit gereinigtem Wasser für einen ausreichenden Zeitraum, um eine Mischung zu bilden;
(ii) Lyophilisieren der Mischung zu einer Kollagenlage; und
(iii) Vernetzen der Kollagenlage.

15. Kollagenartikel nach einem der Ansprüche 1 bis 7, wobei der Kollagenartikel aus der vernetzten Kollagenlage besteht.

## Revendications

1. Dispositif de collagène pour une utilisation comme substitut dural, ledit dispositif de collagène comprenant une feuille de collagène réticulée, ladite feuille ayant une résistance à la traction d'environ 58,6 kPa (8,5 psi) ou plus, ladite feuille ayant une pluralité de pores, une majorité parmi lesdits pores ayant un diamètre inférieur à 10 micromètres.

2. Dispositif de collagène selon la revendication 1, où la résistance à la traction est d'environ 58,6 kPa (8,5 psi).

3. Dispositif de collagène selon la revendication 1 ou 2, où plus de 95% des pores du dispositif possèdent un diamètre inférieur à 10 micromètres.

4. Dispositif de collagène selon la revendication 1 ou 2, où plus de 98% des pores du dispositif possèdent un diamètre inférieur à 10 micromètres.

5. Dispositif de collagène selon la revendication 1 ou 2, où environ tous les pores du dispositif possèdent un diamètre inférieur à 10 micromètres.

6. Dispositif de collagène selon l'une quelconque des revendications précédentes, où le collagène est du collagène recombiné.

7. Dispositif de collagène selon l'une quelconque des revendications précédentes, où le dispositif de collagène contient un agent actif biologique.

8. Dispositif de collagène selon l'une quelconque des revendications précédentes, comprenant en outre, fixé à, ou incorporé dans, la feuille, au moins l'un parmi un film, un feutre, une matrice, un maillage ou une deuxième feuille de collagène.

9. Dispositif de collagène selon la revendication 8, où un film imperméable fluide est fixé à, ou incorporé dans, la feuille.

10. Dispositif de collagène selon la revendication 8 ou 9, où le film, le feutre, la matrice, le maillage ou bien la deuxième feuille de collagène, contient un agent actif biologique.

11. Dispositif de collagène selon la revendication 10, où la première feuille de collagène contient un agent actif biologique, et le film, le feutre, la matrice, le maillage ou la deuxième feuille de collagène, contient également un agent actif biologique, et l'agent actif biologique contenu dans la première feuille est différent de l'agent actif biologique contenu dans le film, le feutre, la matrice, le maillage ou bien la deuxième feuille de collagène.

12. Dispositif de collagène selon la revendication 10, où la première feuille de collagène contient un agent actif biologique, et le film, le feutre, la matrice, le maillage ou la deuxième feuille de collagène, contient également un agent actif biologique, et l'agent actif biologique contenu dans la première feuille est le même que l'agent actif biologique contenu dans le film, le feutre, la matrice, le maillage ou bien la deuxième feuille de collagène.

13. Dispositif de collagène selon l'une quelconque des revendications 7 ou 10-12, où l'agent actif biologique est au moins un facteur de croissance, un antibiotique, ou une combinaison d'antibiotiques, ou un agent anticancéreux ou une combinaison d'agents anticancéreux.

14. Dispositif de collagène selon l'une quelconque des revendications précédentes, où ladite feuille de collagène réticulée peut être obtenue par un procédé comprenant les étapes consistant à :
(i) mélanger du collagène avec de l'eau purifiée pendant une période de temps suffisante afin de former un mélange ;
(ii) lyophiliser le mélange en une feuille de collagène ; et
(iii) réticuler la feuille de collagène.

15. Dispositif de collagène selon l'une quelconque des revendications 1 à 7, où le dispositif de collagène est constitué de ladite feuille de collagène réticulée.
